# EUROPEAN PATENT APPLICATION

(11) **EP 1 744 159 A1**
(43) Date of publication of application: **17.01.2007**
(21) Application number: 04724151.8
(22) Date of filing: 29.03.2004
(51) Int. Cl.: G01N 33/53

(54) **METHOD OF ASSAYING APOLIPOPROTEIN B-48 AND USE THEREOF**

(71) Applicant: Shibayagi Co., Ltd., Shibukawa, Gunma 3770007 (JP)
(72) Inventor: KOJIMA, Masaaki, Shibukawa-shi, Gunma 3770007 (JP)
(74) Representative: Gille Hrabal Struck Neidlein Prop Roos
(86) International application number: PCT/JP2004/004434
(87) International publication number: WO 2005/093415

(57) **Abstract**

The present invention provides a method for the measurement of apoB-48, which involves measuring the apoB-48 by diluting a sample with a tris-buffer and, as needed, a surfactant and reacting the diluted sample with an anti-apoB-48 monoclonal antibody, and a kit for measuring the apoB-48 by using the above method. The method and the kit can accurately, rapidly and readily measure the apoB-48 particularly in blood.

The method for the measurement of the apoB-48 and the kit to be used therefor are applicable to diagnosis of diseases associated with the apoB-48 and assessment of an efficacy of an ingredient of medicine for treating hyperlipidemia and an ingredient of functional food by measuring an increase or decrease of the apoB-48 in blood,

## Description

### Technical Field

The present invention relates to a method for the measurement of apolipoprotein B-48 and its use. More particularly, it relates to a method for the measurement of apolipoprotein B-48 and a kit for the measurement of apolipoprotein B-48 as well as a method for the diagnosis of apolipoprotein B-48-related diseases by using the same and a method for the assessment of efficacy of ingredients of medicine for treating hyperlipoproteinemia increasing or decreasing apolipoprotein B-48 in the blood and ingredients of functional foods by using the same.

### Background Technology

Presently, the numbers of death of the Japanese people caused by heart diseases and cardiovascular diseases, in particular arteriosclerosis, amount to the second ranking next to malignant neoplasm (cancers). Therefore, it is of urgent necessity to decrease the percentage of mortality caused by arteriosclerosis by the prevention and treatment of arteriosclerosis from the point of view of health care for the people in a coming advanced age society.

It can be noted here, however, that in the current situation there is no established methods for surely and effectively performing the diagnosis and treatment of arteriosclerosis so that the development of a sure and effective method for the diagnosis and treatment of arteriosclerosis is a growing demand. The cause of an occurrence of arteriosclerosis is not so uniform and it is made clear that various factors and events including, but not limited to, hyperlipidemia, hypertension, diabetes mellitus, obesity, smoking, heredity and aging are interrelated with each other. Among these factors, hyperlipidemia is considered to be the highest risk factor.

Hyperlipidemia may cause an occurrence of arteriosclerosis in the systemic blood vessels and injuries in various organs as arteriosclerosis develops and the stream of blood becomes worse. As the arteriosclerosis occurs in the cardiac blood vessels (the coronary artery), on the one hand, the ischemic cardiac diseases (i,e., angina pectoris and myocardial infarction) may be caused to occur, As the arteriosclerosis occurs in the cerebral blood vessels, on the other hand, diseases of the cerebral blood vessels (e.g., cerebral infarction) may be caused to occur,

Hyperlipidemia is a state in which lipids in the blood, including, for example, in particular cholesterol and neutral fats (e.g., triglycerides) are present in excessive amounts, Cholesterol and triglycerides (neutral fats) are not dissolved in the blood as they are intact, because the most portion of the blood is occupied by water. Therefore, cholesterol and triglycerides are dissolved in the blood in the form of globular particles which consist of lipoproteins. The lipoproteins are composed of hydrophilic phospholipids, free cholesterol and apoproteins in the outermost layer as well as hydrophobic cholesterol (mostly ester-type cholesterol) and triglycerides (neutral fats) in the central portion, i.e., core layer. These lipids constitute cell membranes in the body and originally play a significant role as materials for steroid hormones and the like. If the lipids, particularly cholesterol and triglycerides, are present in excessive amounts in the blood, however, they may cause hyperlipidemia.

As arteriosclerosis is caused to occur due to hyperlipidemia or for other reasons, various lipoproteins involved in the acceleration or inhibition of deposition of cholesterol onto the inner walls of the blood vessels, among the lipoproteins composed of cholesterol and triglycerides (neutral fats) present in the blood circulating through the body, may be increased or decreased, Therefore, an accurate measurement of the amounts of these lipoproteins in the blood can assist in the diagnosis and treatment of hyperlipidemia and arteriosclerosis.

It is to be noted herein, however, that the lipoproteins in the blood are in the form in which the apolipoproteins are connected to serum lipids and they play a role as a carrier of serum lipids.

The lipoproteins have different densities and particle sizes by their lipid compositions and are generally divided by different densities and particle sizes into five categories, i.e., chylomicrons (CM), very low density lipoproteins (VLDL), intermediate density lipoproteins (IDL), low density lipoproteins (LDL), and high density lipoproteins (HDL). These lipoproteins have from lower density levels to higher density levels and from larger particle sizes to lower particle sizes in this order. It can also be noted that these lipoproteins are composed of cholesterol and neutral fats (triglycerides: TG) at different ratios and that particles having larger sizes contain neutral fats at larger ratios than smaller ones while particles having smaller sizes contain cholesterol at larger ratios than larger ones.

Among these lipoproteins, the chylomicrons (CM) are primarily composed of triglycerides (neutral fats) and are synthesized by the small intestine from triglycerides and cholesterol derived from meal by the small intestine and secreted into the blood stream. The chylomicrons are secreted into the blood stream via the lymphoduct and the triglycerides are decomposed by lipoprotein lipase (LPL) into chylomicron remnants (CM-R) which have smaller particle sizes and are rich in cholesterol. The chylomicron remnants are then taken up by the liver through apolipoprotein E receptor. As described above, the chylomicrons (CM) play a role in transporting exogenous triglycerides (originating from meal) into the adipose tissues and the muscle and at the same time carrying cholesterol to the liver.

The very low density lipoproteins (VLDL) are composed primarily of triglycerides (neutral fats) and created by the liver and small intestine and secreted therefrom. Then, the very low density lipoproteins transport mainly endogenous lipids, namely, endogenous triglycerides and endogenous cholesterol synthesized by the liver cells, from the liver into the blood stream, Although a portion of the VLDL is absorbed directly into the tissues, the most portion thereof allows the triglycerides to be decomposed by the lipoprotein lipase (LPL) into the intermediate density lipoproteins (IDL = VLDL remnants).

The intermediate density lipoproteins (IDL) are then absorbed into the liver via apolipoprotein E and decomposed by the lipase into low density lipoproteins (LDL).

AS described nabove, the low density lipoproteins (LDL) are produced as a result of the metabolism of the very low density lipoproteins (VLDL) through the intermediate density lipoproteins (IDL), The LDL contains cholesterol in the amount larger than any other lipoproteins as well as is composed of approximately 40% of cholesterol which is mainly supplied into the liver and almost all tissues. As the LDL is small in particle size, it has the property prone to move through the arterial wall. The LDL passed through the arterial wall becomes very likely to be oxidized, unlike in the blood, into oxidized LDL that in turn acts as a trigger for causing an occurrence of arteriosclerosis. In order to prevent and treat the arteriosclerosis, therefore, it is considered to be of great importance to decrease the amount of cholesterol as well as protect from oxidation.

On the other hand, the high density lipoproteins (HDL) are mainly produced by the small intestine and liver, although they are formed even during the steps of catabolism of the chylomicrons and the very low density lipoproteins (VLDL) by an action of the lipoprotein lipase. The HDL plays a role in conducting catabolism by collecting an excessive amount of cholesterol in the cells of the peripheral tissues and cholesterol accumulated in the arterial wall and transporting them to the liver, Therefore, it is recently considered that an increase of the amount of the HDL can lead to an improvement of hyperlipidemia and finally to prevention and treatment of arteriosclerosis.

Most recently, however, it is becoming evident that a measurement for the lipid levels at the time of hunger is not sufficient in order to assess a risk of arteriosclerosis, and an assessment for a risk of arteriosclerosis by the lipid levels after meal, that is, an assessment of hyperlipidemia after meal, is considered to be needed.

Lipids which increase after meal are triglycerides (TG). Hypertriglyceridemia is caused to occur by an increase of lipoproteins rich in TG, that is, TG-rich lipoproteins including chylomicrons (CM), very low density lipoproteins (VLDL) and remnant lipoproteins as metabolic products therefrom, Among the three lipoproteins causing hypertriglyceridemia, the remnant lipoproteins are considered to be most important for the assessment of a risk of arteriosclerosis.

In other words, the chylomicrons containing a rich amount of exogenous TG derived from meal and produced in the small intestine are decomposed by the lipoprotein lipase (LPL) into the CM remnant that in turn taken up by a remnant receptor recognizing the apolipoprotein E and treated by the liver.

It is reported that there is presently no simple, reproducible and rapid assay method for distinguishing between conjugates of exogenous lipids derived from meal in the circulation, that is, chylomicrons and chylomicron remnants, and conjugates of endogenous secreted from the liver, that is, very low density lipoproteins (VLDL) and low density lipoproteins (LDL) (Lovegrove, J. A., et al.: Biochim. et Biophy. Acta, 1301 (1996) 221-229),

It can be noted herein, however, that no great variation in the chylomicrons (CM) and very low density lipoproteins (VLDL) before and after meal is recognized, on the one hand, while the remnant lipoproteins, that is, the CM remnants and the VLDL remnants, which are metabolic products of the chylomicrons and the very low density lipoproteinss, formed by the decomposition and metabolism of the triglycerides by an action of the lipoprotein lipase (LPL), are increased after meal, but rapidly absorbed and decomposed by the liver cells and so on for healthy people, on the other hand, and they exist no longer in the blood at the time of hunger, The metabolism of these remnant lipoproteins, however, may become slowed down due to abnormality of the metabolism, overeating, a lack of exercise and the like. It is now considered that, if they would stay in the blood after meal, they are readily absorbed by macrophages in the arterial wall leading to a cause of the initial lesion of arteriosclerosis. The remnant lipoproteins are called remnant-like particles (RLP) and it is also considered that they are a significant factor associated with hyperlipidemia after meal.

On the other hand, there exist apolipoproteins (apoproteins) as protein components constituting the remnant-like lipoproteins. The apolipoproteins have a role in transporting the lipids into the blood in the form likely to be dissolved in the blood by conjugating to the water-insoluble lipids (ester-type cholesterol, neutral fats and so on) and they are also involved deeply in the metabolism of various lipids, As such apoproteins, there are presently confirmed more than ten apolipoproteins which include, but are not limited to, apoprotein A-I, apoproteins A-II, apoproteins B, apoproteins C-I, apoproteins C-II and apoproteins E.

Among the apoproteins, the apoproteins B is further broken down into two apoproteins, i.e., apoprotein B-100 (apoB-100) and apoprotein B-48 (apoB-48), The former on the one hand is a major protein component of the human plasma VLDL synthesized by the liver and plays an important role in its synthesis and secretion, and the latter on the other is produced by the small intestine and involved in the synthesis of the chylomicrons (CM). The apoB-100 amounts to approximately 40% to 60% of the proteins present in the very low density lipoproteins and to approximately 98% of the low density lipoproteins (LDL) (Mahley, R. W., et al: J. (1984) Lipid Res. 25, 1277-1294). The apoB-48 is a protein constituting the lipoproteins associated closely with hyperlipidemia in the human blood. Therefore, an accurate measurement for the apoB-48 is useful for the diagnosis and treatment of hyperlipidemia and arteriosclerosis.

It is very difficult, however, to accurately distinguish the apoB-48 from the apoB-100 because the apoB-48 is identical to the amino acid sequence of approximately 48% of the N-terminal amino acid sequence of the apoB-100 and has a very high homology to the apoB-100 (Lovegrove, J. A., et al.: ibid).

As described above, this remnant lipoprotein has been known to be a risk factor for arteriosclerosis, however, it has not been actually used for clinical examinations for a long time because its measurement is not easy. The methods for the measurement of the apoB-48 which have conventionally been used so far include, among others, ultracentrifugation method separating by density differentials, agarose electrophoresis separating by charges, electrophoresis separating by a difference in sizes, and method for the measurement of lipoproteins by using an antibody to the apoprotein. These conventional methods, however, are less practical in actual application because, for example, they are each difficult to measure, they are each lack in quantitative assay, or they are each less specific.

As it is also very difficult to accurately measure the remnant-like lipoproteins (RLP), a method is developed which involves measuring the concentration of the remnant-like lipoproteins (RLP) in the blood as a concentration of cholesterol present in the remnant-like lipoproteins, that is, remnant-like particles-cholesterol (RLP-C).

The method for the measurement of the RLP-C is a measurement method using a monoclonal antibody to apoA-1 and a monoclonal antibody to apoB-100, which comprises removing the apoA-I and the apoB-100 by conjugation with the respective monoclonal antibodies and measuring an amount of non-conjugate lipoproteins remaining in a supernatant as an amount of cholesterol. More specifically, this method allows the apoA-1 to be removed due to a conjugation of the CM and HDL to the monoclonal antibody to apoA-1 because the apoA-1 is present as a major apoprotein of the CM and HDL, while it allows the apoB-100 to be removed due to a conjugation of the nascent (wild-type) VLDL (or VLDL-2) and the LDL to the monoclonal antibody to apoB-100 because the monoclonal antibody to apoB-100 is specific to a particular amino acid domain of the apoB-100 but it does not recognize the apoB-48 having a partial amino acid sequence of the apoB-100. Therefore, this method can measure the apoB-48-containing the chylomicrons and apoE-rich VLDL.

The method for the measurement of the RLP-C, however, requires a step for removal by adsorption so that its work is laborious and complex as well as it requires experienced skills for carrying out measurement operations because a thorough adsorption is required.

As described hereinabove, the apoB-48 is contained in the CM and CM remnant as apoproteins. As the CM is rapidly converted to the CM remnants after meal by an action of the lipoprotein lipase, it does not exist in the blood any longer at the time of hunger. If it would be possible to measure the amount of only the apoB-48 contained in the CM remnants, it is considered that a secure diagnosis of hyperlipidemia may become feasible and this can greatly assist in the diagnosis and treatment of arteriosclerosis.

In addition, it can be considered that an amount of only the CM remnants present in the blood can be measured directly if a monoclonal antibody to apoB-48 capable of recognizing specifically to the apoB-48 alone could be developed.

As one of these measurement methods, there is reported an ELISA method using anti apoB-48-monoclonal antibody for quantitatively diagnosing hyperlipidemia after meal (Kinoshita, M., et al; "Development of Method for Measurement of ApoB-48-containing Lipoprotein", Abstracts of The 30th General Convention of The Japan Arteriosclerosis Society, page 133, June 11-12, 1999). This method comprises preparing a monoclonal antibody (4C8) capable of specifically recognizing the apoB-48, immobilizing the monoclonal antibody to a plate, adding serum diluted with PBS containing 2% SDS to the plate, and measuring the apoB-48-containing lipoproteins. There is disclosed, however, that no apoB-48 in serum can be measured in 2% SDS and, conversely, SDS inhibits the immune reaction so that the use of SDS is not advisable to be used as a surfactant for an exposure of the epitope of the apoB-48 (Japanese Patent No. 3,440,852, col. 8, pp. 7-9).

As another method for measuring the apoB-48 using a monoclonal antibody to apoB-48, there is disclosed an immunoassay method for measuring apoB-48 and/or apoB-48-containing lipoproteins, which is characterized in treating a sample with a surfactant selected from a non-ionic surfactant and an anionic surfactant, i.e., sodium deoxycholate, or freezing and thawing the sample in order to allow an exposure of an epitope of the apoB-48 and then reacting the sample with a monoclonal antibody recognizing specifically the cpoB-48 but not substantially reacting with the apoB-100 (Japanese Patent Application Publication No. 2003-270,427A),

The method as disclosed in the above patent involves determining an exposure of the C-terminal epitope of the apoB-48 by diluting serum with PBS containing the various surfactants and adding the monoclonal antibody specifically recognizing the opoB-48 to the diluted serum (ibid., col, 12, II. 30-38), In the case where the non-ionic surfactants such as Triton™X-100, Triton™X-114, Tween-20™, NP™ -40 and the like are used, a strong color development can be recognized and the exposure of the C-terminal epitope of the apoB-48 is determined, while no color development is observed in the case where no surfactant is used (ibid., col. 14, II, 12-26),

In order to develop a new method capable of accurately measuring the apoB-48, the present inventors have extensively studied improvements in the methods for measuring the apoB-48 and, as a result , they have found a method for measuring the apoB-48, which can accurately measure the apoB-48. The present invention is based on this finding.

### Disclosure of the Invention

Therefore, the present invention has the object to provide a method for the measurement of an apolipoprotein B-48, which does not necessarily require the use of any surfactant and comprises measuring the apoB-48 by applying a monoclonal antibody to apoB-48 to a sample diluted with a tris-buffer solution.

The present invention also has the object to provide a kit for measuring the apoB-48 which can be applied to the above method for the measurement of the apoB-48 according to the present invention.

The present invention in its preferred embodiment has the object to provide a method for the measurement of the apoB-48 and a kit for measuring the apoB-48, which further comprises each using a surfactant.

Furthermore, the present invention has the object to provide a method for the diagnosis or treatment of various diseases associated with the apoB-48 by using the above method and kit for the measurement of the apoB-48 according to the present invention as well as measuring the apoB-48 accurately, rapidly and simply.

In addition, the present invention has another object to provide a method for assessing an efficacy of an ingredient of medicine for curing hyperlipidemia and an ingredient of functional food by using the kit according to the present invention.

### Best Modes for carrying out the Invention

The method for the measurement of the apoB-48 according to the present invention comprises measuring the apoB-48 in a dilution of a sample diluted with a tris-buffer solution by using a monoclonal antibody to apoB-48,

As the monoclonal antibodies to the apoB-48 to be used for the present invention, there may be used any anti-apoB-48 monoclonal antibody that can specifically recognize a portion of the amino acid sequence of the apoB-48 from the C-terminus thereof, and they may include, but not be limited to, anti-apoB-48 monoclonal antibody (4C8) as described above. Such monoclonal antibodies may be prepared by conventional methods as known to the art.

As the tris-buffer solution to be used for the present invention, there may be used any one as long as it can achieve the objects of the present invention, and it may include, but not be limited to, tris(2-amino-2-hydroxymethyl-1 ,3-propanediol-tris), tris-HCl buffer [tris(hydroxymethy)aminomethane hydrochloride (pH 7.2-9.0)], and tris-maleic acid buffer [tris(hydroxymethyl)aminomethane maleate (pH 5.2-8.6)]. The tris-buffer to be used herein may be cloassified as Good's buffer, and may also include, but not be limited to, MES, Bis-tris, ADA, Bis-tris propane, PIPES, ACES, MOPSO, cholamine chloride, BES, MOPS, TES, HEPES, DIPSO, TAPSO, POPSO, HEPPSO, HEPPS(EPPS), Tricine, Bicine, and glycine amide.

The concentration of the tris-buffer solution to be used in the present invention may appropriately vary with kind and so on and may be, for example, in the range of from 10 mM to 0.5 M in the case of tris-HCl buffer solution.

For the method for the measurement of the apoB-48 according to the present invention, it is not required to use a surfactant, although it is possible to add a surfactant as needed, Such a surfactant may include, but not be limited to, Triton X-100™, Triton X-114 ™, Tween-20 ™ and NP-40 ™, These surfactants may be used singly or in combination with the other.

The surfactant is used to make it likely to allow an exposure of the epitope of a lipoprotein, but it generally inhibits an immune reaction. By taking a balance between the effect of exposing the epitope of a lipoprotein and the action of inhibition of the immune reaction, however, the concentration of the surfactant may appropriately be selected. The surfactant may be used in a solution at a concentration of generally from 0.01% to 2%, preferably from 0.02% to 0,5%, upon carrying out the immune reaction of the sample with a monoclonal antibody. Although the temperature and period of time of treatment of the surfactant are not limited to particular ones, the temperature may range generally from 4 °C to 40 °C and the period of time may range generally from approximately 5 minutes to 48 hours.

For the present invention, the epitope of the lipoprotein can be exposed by repeating steps of freezing and thawing the sample so that the steps of freezing and thawing the sample are repeated preferably more than one time.

The method for the measurement of the apoB-48 in accordance with the present invention will be described hereinafter in more detail, It is to be understood herein, however, that the following description is made with the intent not to limit the scope of the present invention in any respects and with the object to illustrate the invention in a more specific fashion. Therefore, the present invention is not to be understood that it is limited in any means by the description which follows.

The method for the measurement of the apoB-48 according to the present invention comprises, for example, diluting a sample with a tris-buffer solution, adding the diluted sample to a plate with a purified IgG product of the anti-apoB-48 monoclonal antibody (4C8) immobilized thereon to allow the apoB-48 contained in the sample to be conjugated thereto, conjugating a labeled anti-apoB-48-/apoB-100-biotin-labeled antibody and then an avidin-conjugated horse radish peroxidase (HRP) to the apoB-48, and measuring the apoB-48 by means of ELISA assay.

In the method for the measurement of the apoB-48 according to the present invention, first, a serum sample is prepared by adding, for example, 0,1M tris-HCl buffer (pH 7.8) to serum as a sample and the serum sample is then added to a 96-well plate with anti-opoB-48 monoclonal antibody (4C8) IgG immobilized thereon, followed by the reaction of the serum sample with the monoclonal antibody for a predetermined period of time in order to conjugate the apoB-48 in the serum sample to the anti-apoB-48-monoclonal antibody (4C8). Thereafter, an anti-apoB-48/B-100-biotin labeled antibody is added and reacted for a predetermined period of time to allow a conjugation of the above labeled antibody. The labeled antibody is then reacted with an avidin-conjugated peroxidase such as avidin-conjugated HRP or the like to permit conjugation to the labeled antibody, followed by reaction with a developing substrate such as tetramethyl benzidine (TMB) or the like and termination of the reaction with a reaction terminator such as sulfuric acid or the like. The sample is then assayed for absorbency at 450 nm,

The method for the measurement of the apoB-48 according to the present invention is preferably carried out by immunoassay methods (methods utilizing the principle of an antigen-antibody reaction) such as ELISA or the like, although other immunoassay methods can also be used. For example, this method can be applied to the non-labeled sedimentation method (turbimetric analysis, nephelometric analysis, etc.), immobilization method (latex method, surface plasmon resonance method, quartz crystal oscillation method, etc.), labeled measurement methods (radioimmunoassay, enzyme-linked immunoassay, luminescence immunoassay, fluorescence immunoassay, metal-labeled immunoassay, etc.),

The present invention in another embodiment is a kit for the measurement of apoB-48 which comprises an anti-apoB-48 monoclonal antibody including, but being not limited to, the apoB-48-monoclonal antibody (4CB), a tris-buffer solution including, but not being limited to, a tris-HCl buffer solution, the apoB-48/apoB-100-biotin-labeled antibody, an avidin-conjugated peroxidase including, but being not limited to, avidin-conjugated horse radish peroxidate (HRP), a developing substrate including, but being not limited to, tetramethyl benzidine (TMB), and a reaction terminator including, but being not limited to, sulfuric acid. As the anti-apoB-48-monoclonal antibody, there may be used anti-apoB-48-monoclonal antibody (4C8) IgG immobilized on a 96-well plate.

The present invention will be described in more detail by way of examples, It is to be understood that, on the one hand, the present invention is not limited to the following examples and, on the other hand, the following examples are not construed in any means as being described for limiting the present invention and they are described with the object solely to specifically illustrate the present invention,

### Example 1:

As a sample to be measured was used serum to which 0,1M tris-HCl (pH 7,8) is added. A 96-well plate with anti-apoB-48-monoclonal antibody (4C8) IgG immobilized thereon was washed three times with a washing buffer solution. To the plate was added 50 µl/well of an apoB-48 standard preparation (1 60 ng/ml -2,5 ng/ml) and 50 µl/well of the measuring sample to be measured. The resulting plate was then allowed to react at room temperature for 1 hour and to conjugate the apoB-48 in the sample to the apoB-48 monoclonal antibody (4C8), followed by washing the plate three times with the washing buffer solution. Thereafter, 50 µl per well of the reaction product was mixed with 50 µl per well of an anti-apoB-48-/B-100-biotin-labeled antibody at a rate of and reacted at room temperature for 1 hour to conjugate the labeled antibody, followed by washing the resulting reaction mixture three times with the washing buffer. To 50 µl per well of the reaction product was then added 50 µl per well of avidin-conjugaied HRP to conjugate the avidin-conjugated HRP to the labeled antibody, and 50 µl per well of tetramethyl benzidine (TMB) was added and the resulting mixture was reacted at room temperature for 30 minutes, followed by an addition of 1 M sulfuric acid in an amount of 50 µl per well to terminate the reaction and a measurement of absorbency at 450 nm. The result is shown in Table 1 .

### Example 2:

A serum sample to be measured was prepared by adding 1 M tris-HCl solution (pH 7.8) containing 0.1% Triton^{™} X-1 00 to serum. Separately, a 96-well plate for immobilizing anti-apoB-48 monoclonal antibody (4CB) IgG was washed three times with a washing buffer solution and each well of the plate was immobilized with 50 µl/well of an apoB-48 standard product (160 ng/ml - 2.5 ng/ml). To each well was added 50 µl of the serum sample to be measured. The plate was allowed to react at room temperature for 1 hour to conjugate the apoB-48 in the sample to the anti-apoB-48 monoclonal antibody (4C8), followed by washing the wells of the plate three times with a washing buffer solution. To 50 µl/well of the resulting reaction product was added 50 µl/well of anti-apoB-48-/apoB- 100-biotin-labeled antibody, and the reaction product was then reacted at room temperature for 1 hour to allow the reaction product to conjugate to the labeled antibody. Thereafter, the resulting conjugate was washed three times with the washing buffer solution, followed by adding 50 µl/well of avidin-conjugated HRP to each well and reacting the resulting mixture at room temperature for 1 hour to conjugate to the labeled antibody. To each well was then added 50 µl/well of tetramethyl benzidine (TMB) and the resulting mixture was reacted at room temperature for 1 hour. Then, 50 µl of 1M sulfuric acid was added to each well to terminate the reaction and the resulting mixture was measured for absorbency at 520 nm, The measurement result is shown in Table 1,

**Table 1 :**

| Buffer | 0,1M Tris-HCl (pH7,8) | 0.1% Triton X-100-containing 0,1M tris-HCl (pH7.8) |
|---|---|---|
| Average | 1,64 µg/mg | 1,70 µg/ml |

### Example 3: Kit

A kit for measuring apoB-48 according to the present invention in another embodiment comprises the components as follows:
96-well plate with anti-apa-B48-monoclonal antibody (4C8) IgG immobilized thereon,
apoB-48 standard product (antigen for preparing calibration line),
tris-HCl buffer solution,
anti-apoB-48/apoB- 100-biotin-labeled antibody,
avidin-conjugated horse radish peroxidase (HRP),
tetramethyl benzidine (TMB),
sulfuric acid, and
concentrating and washing solution.

### Example 4:

An amount of apoB-48 of a healthy person at the time of hunger was measured in substantially the same manner as in Example 1, The measurement result is shown in Table 2.

**Table 2:**

| | ApoB-48 (µg/ml) |
|---|---|
| Average | 4,56 |

### Example 5:

An amount of apoB-48 of a person died suddenly was measured in substantially the same manner as in Example 1 , The measurement result is shown in Table 3,

**Table 3:**

| | ApoB-48 (µg/ml) |
|---|---|
| Average | 17.60 |

### Example 6:

An amount of apoB-48 in a blood sample of a person with acute myocardial infarction was measured in substantially the same manner as in Example 1, The measurement result is shown in Table 4,

**Table 4:**

| | ApoB-48 (µg/ml) |
|---|---|
| Average | 30.97 |

### Industrial Applicability

The method for the measurement of the apoB-48 according to the present invention can measure the apoB-48 in a sample so accurately, rapidly and readily that it is useful for the diagnosis and treatment of diseases such as hyperlipidemia and asteriosclerosis. The method according to the present invention can measure the apoB-48 in blood with accuracy and certainty so that it is efficient for checking diseases associated with coronary arteries and it is further of assistance in preventing sudden death.

In addition, the present invention can be applied to assess en efficacy of ingredients of medicine for treating hyperlipidemia and ingredients of functional food by measuring an increase or decrease of the apoB-48 in blood.

## Claims

1. A method for the measurement of apoB-48 comprising diluting a sample with a tris-buffer, reacting the diluted sample with an anti-apoB-48 monoclonal antibody and measuring the apoB-48.

2. The method for the measurement of apoB-48 as described in Claim 1, comprising reacting said diluted sample with said anti-apoB-48 monoclonal antibody to conjugate the apoB-48 in the sample to said anti-apoB-48 monoclonal antibody, adding an anti-apoB-48/apoB-100-biotin-labeled antibody to conjugate the anti-apoB-48/apoB-100-biotin-labeled antibody to the apoB-48, reacting an avidin-conjugated peroxidase to yield a conjugated product, reacting the resulting conjugated product with a developing substrate, and then measuring the apoB-48,

3. The method for the measurement of apoB-48 as described in Claim 1 or 2, wherein said tris-buffer is tris, tris-HCl buffer, tris-maleate buffer or Good's buffer.

4. The method for the measurement of apoB-48 as described in any one of Claims 1 to 3, wherein said diluted sample is further mixed with a surfactant.

5. The method for the measurement of apoB-48 as described in any one of Claims 1 to 4, wherein said surfactant is a non-ionic surfactant,

6. A kit for measuring apoB-48 comprising a fris-buffer and an anti-apoB-48 monoclonal antibody.

7. The kit for measuring apoB-48 as described in Claim 6, further comprising an anti-apoB-48/apoB-100-biotin-conjugated anitbody, an avidin-conjugated peroxidase and a developing substrate.

8. The kit for measuring apoB-48 as described in Claim 6 or 7, wherein said tris-buffer comprises tris, tris-HCl buffer, tris-maleate buffer or Good's buffer.

9. The kit for measuring apoB-48 as described in any one of Claims 6 to 8, further comprising a surfactant.

10. The kit for measuring apoB-48 as described in any one of Claims 6 to 9, wherein said surfactant is a non-ionic surfactant,

11. A method for the measurement of apoB-48 comprising measuring the apoB-48 in a sample by using the method for the measurement of the apoB-48 as described in any one of Claims 1 to 5 and/or the kit for measuring the apoB-48 as described in any one of Claims 6 to 10.

12. A method for diagnosis of disease associated with apoB-48 comprising measuring the apoB-48 in a sample by using the method for the measurement of the apoB-48 as described in any one of Claims 1 to 5 and 11 and/or the kit for measuring the apoB-48 as described in any one of Claims 6 to 10.

13. The method for diagnosis as described in Claim 12, wherein said disease associated with apoB-48 is hyperlipidemia, asteriosclerosis, cardiac insufficiency, or sudden death,

14. A method for assessing an efficacy of an ingredient of medicine for treating hyperlipidemia or an ingredient of functional food, comprising using the method for the measurement of the apoB-48 as described in any one of Claims 1 to 5 and 11 and/or the kit for measuring the apoB-48 as described in any one of Claims 6 to 10,

15. A method for analysis of disease **characterized by** measuring multiple items in an identical sample with an identical measuring device by combining the method for the measurement of the apoB-48 as described in any one of Claims 1 to 5 with a measuring item or items other than the apoB-48,
